# EUROPÄISCHE PATENTSCHRIFT

(11) **EP 0 562 303 B1**
(45) Veröffentlichungstag und Bekanntmachung des Hinweises auf die Patenterteilung: **16.08.1995**
(21) Anmeldenummer: 93103192.6
(22) Anmeldetag: 27.02.1993
(51) Int. Cl.: B01D 67/00, B01D 69/02

(54) **Chemisches Ventil**
Chemical valve
Vanne chimique

(30) Priorität: 21.03.1992 DE 4209264
(43) Veröffentlichungstag der Anmeldung: 29.09.1993
(73) Patentinhaber: Gesellschaft für Schwerionenforschung mbH, D-64291 Darmstadt (DE)
(72) Erfinder: Spohr, Reimar, Dr., W-6100 Darmstadt (DE); Tamada, Masao, Takasaki, Gunma-ken 370-12 (JP); Omichi, Hideki, Takasaki, Gunma-ken 370-12 (JP); Trautmann, Christine, W-6100 Darmstadt (DE); Vetter, Johann, W-6100 Darmstadt (DE); Yoshida, Masaru, Takasaki, Gunma-Ken 370-12 (JP)
(74) Vertreter: Rückert, Friedrich, Dr.

(56) Entgegenhaltungen:
- DE-A- 2 319 101
- GB-A- 2 224 668

## Beschreibung

Die vorliegende Erfindung betrifft ein chemisches Ventil mit einer porösen Matrix sowie einer durch ihre Umgebungsbedingungen stimuliert quellbaren Gelschicht, deren dadurch bedingte Kontraktionen oder Dilatationen die Poren in der Matrix öffnen oder verschließen, sowie ein Verfahren zur Erzeugung eines solchen Ventiles.

Als chemische Ventile bezeichnet man poröse Membranen,deren Porositäts- und Durchlasseigenschaften durch im allgemeinsten Sinn chemische oder auch thermisch-physikalische Prozesse gesteuert werden können. Aus der GB 22 24 668 A ist z.B. ein flüssigkeitsdurchlässiges Filter bekannt, bei welchem eine Gelschicht auf eine durchlässige Matrix aufgebracht ist. Das Filter bleibt dabei immer durchlässig. Es werden auch auf poröse Substrate oder dreidimensionale Netzwerke polymerisierende Materialien aufgebracht, deren Struktur z.B. durch Ändern des pH-Wertes der durchgeleiteten Flüssigkeit veränderbar ist. Ein Ziel solcher Ventile aus selbstregelnden, "intelligenten" Materialien ist z.B die kontrollierte Abgabe von Medikamenten im Körper. Man kann mit diesen Materialien die Wirkung biologischer Membranen erzielen.

Die bekannten chemischen Ventile weisen jedoch aufgrund ihres Herstellungsprozeßes eine sehr breite Porengrößenverteilung und damit eine sehr unregelmäßige Struktur auf, die sich negativ auf die Kontrolle der Sperrfunktion auswirkt.

Ausgehend von diesem Stand der Technik hat daher die vorliegende Erfindung zur Aufgabe, ein in der Sperrfunktion verbessertes chemisches Ventil mit genauer definierbarer Durchlässigkeit und Porengröße zu schaffen, sowie ein Herstellungsverfahren für ein solches anzugeben.

Zur Lösung dieser Aufgabe schlägt die vorliegende Erfindung für das chemische Ventil selbst die Merkmale vor, die im kennzeichnenden Teil des Patentanspruches 1 angegeben sind. Weitere, besonders vorteilhafte Ausbildungen des Ventiles sind erfindunggemäß in den Merkmalen der Unteransprüche 2 bis 10 zu sehen. Zur Lösung der Aufgabe bezüglich des Herstellungsverfahrens werden nach der Erfindung die Merkmale der Unteransprüche 11 bis 14 vorgeschlagen.

Das erfindunggemäße Ventil weist nun als besonderen Vorteil Poren mit exakt voraussagbarem, gleichmäßigem Durchmesser in der Matrix auf. Dadurch wird die Sperrfunktion des Ventiles besser definiert, d.h. seine Vorteile bestehen vor allem in einer verbesserten Kontrolle der Membranparameter, dem verbesserten Ansprechen der Membran auf die externen Stimuli, wobei die Steuerung aus dem Arbeitsmedium selbst erfolgen kann.

Einzelheiten des neuen, erfindungsgemäßen chemischen Ventiles und des Verfahrens zu seiner Erzeugung werden im folgenden und anhand zweier schematischer Figuren näher erläutert:

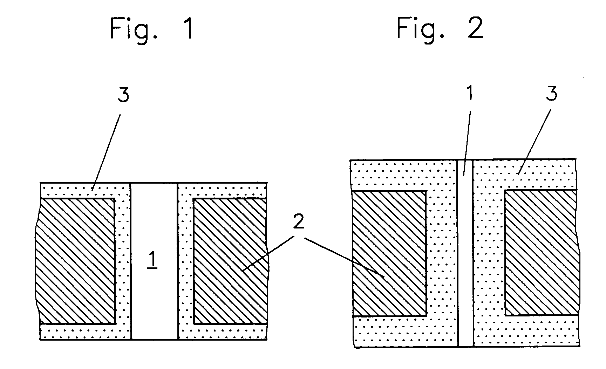

Die Figuren zeigen eine der steuerbaren Poren 1 in einer Folie bzw. Copolymer-oder Polymerschicht 2, die eine responsiv quellbare Oberfläche 3 aufweist. Links, in der Fig.1, ist die Pore 1 in ihrem Ausgangszustand mit einer sich inseitig erstreckenden Gelschicht 3 und rechts, in der Fig.2,-durch ein Stimulans angeregt-im gequollenen Zustand dargestellt. Dieses Aufquellen kann bis zum vollständigen Verschließen der Pore 1 erfolgen. Die Erzeugung der Schicht 3 erfolgt auf verschiedene, später näher beschriebene Weise.

In der weiteren Beschreibung werden nun die folgenden Begriffe bzw. Bezeichnungen verwendet: Unter einem Monomer werden das Ausgangsmaterial bzw. die Moleküle oder -gruppen für Polymerisationsprozesse verstanden, die Moleküle liegen gewöhnlich in flüssiger Form oder als Gas vor. Polymere sind polymerisierte Monomere aus einem Monomer. Unter Copolymeren sind Stoffe zu verstehen, die aus mehreren gemischten oder geschichteten Monomeren polymerisiert sind.

Ausgangsmaterial für die Folie 2 ist ein membranförmiges Copolymer aus zwei oder mehreren Monomertypen mit verschiedenen Eigenschaften, wie z.B Polycarbonat, Polyethylen-Terephthalat oder ein Polyimid wie Kapton (Handelsmarke DuPont). Ein Monomertyp A davon sollte gute Kernspureigenschaften bezüglich der Bildung von latenten Kernspuren nach einer Bestrahlung mit vorzugsweise schweren Ionen aufweisen, d.h. bei einer entsprechenden Bestrahlung identifizierbare Kernspuren erzeugen. Ein weiterer Monomertyp B sollte gute Quwelleigenschaften bei Anregung durch ein Stimulans aufweisen, wobei als solches z.B. die Temperatur, der pH-Wert einer wässrigen Lösung oder eine andere chemische oder physikalische Größe in Frage kommen.

Als Beispiel für ein temperaturabhängiges Material (Monomer A) wird ein Copolymerfilm aus Diethyleneglykol-bis-allylcarbonat als Material mit besonders guten Kernspureigenschaften genannt. Als gut geeignetes Hydrogel, d.h. als Material mit besonders guten Quwelleigenschaften in wässriger Lösung (Monomer B) wird ein aminosäurehaltiges Monomer wie z.B. Methacryloyl-L-alaninmethylester genannt. Die Monomere A und B können nun mehr oder weniger homogen gemischt, oder schichtweise zusammengefügt sein. Günstig ist in diesem Fall eine Schicht des Polymeres A , die wahlweise von verschiedenen Seiten her mit dem Typ B beschichtet sein kann. Bei der Mischung hat sich ein Mischungsverhältnis von A zu B wie 80:20 als besonders vorteilhaft herausgestellt.

Wie bereits erwähnt besitzt das chemische Ventil eine poröse Matrix 2 sowie eine durch ihre beeinflußbaren Umgebungsbedingungen stimuliert quellbare Gelschicht 3, deren Kontraktionen die Poren 1 in der Matrix 2 gesteuert öffnen oder verschließen. Ein ganz wesentlicher Punkt ist nun, daß die Matrix 2 eine Mikroporenmembran mit einer oder mehreren, auf vorbestimmten Durchmesser als Mikroporen 1 aufgeätzten und durch die Matrix 2 durchgehenden parallelen Kernspuren vorzugsweise schwerer Ionen ist. Sie besteht aus einem Copolymer, gebildet aus zwei oder mehreren verschiedenen Monomertypen, wobei, wie bereits oben angeführt, mindestens ein Typ A gute Kernspureigenschaften bezüglich der Bildung von latenten Kernspuren nach einer Bestrahlung mit beschleunigten Teilchen und mindestens ein weiterer Typ B gute Quwelleigenschaften in Abhängigkeit von der Temperatur und/oder von den Bedingungen in einer wässrigen Lösung oder durch ein anderes Stimulans aufweist.

Eine erste Ausführung des chemischen Ventiles besteht nun darin, daß die einzelnen Monomertypen A und B der Matrix 2 miteinander homogen gemischt sind und eine zweite darin, daß einzelne Polymere aus den Typen A und B schichtweise übereinander aufgebracht oder angeordnet sind. Bei dieser zweiten Ausführung wird der Typ B erst nach der Bestrahlung und Aufätzung des Polymeres A in der Monomerphase an dieses angeheftet oder aufpolymerisiert. Die Mikroporen der aufgeätzten Kernspuren gehen somit nur durch das Polymer A hindurch und werden erst später mit dem Monomer B "ausgekleidet". Bei beiden Ausführungen können zusätzlich noch auf oder in der Außenfläche des Polymers B weitere, chemisch oder thermisch stimulierbare Gruppen von Molekülen angeheftet sein.

### Beispiel für die Herstellung eines chemischen Ventiles nach der ersten Ausführung:

Der Polymerfilm des aus den Monomeren A und B gemischten, flüssigen Materiales wird durch Polymerisation in einer Form unter Verwendung von ca. 3% Benzoylperoxid als Katalysator hergestellt. Die gewählte Mischung von Monomer A und B, einschließlich des Katalysators wird in die Form eingegossen. Die Form besteht aus zwei durch einen 100»m dicken Abstandshalter aus Polyethylenterephthalat getrennten Glasplatten. Nach 24h Polymerisation bei einer Temperatur von 75°C werden die Glasplatten vom polymerisierten Film getrennt. Bei der Polymerisation werden Kohlenstoff-Doppelbindungen in Einfachbindungen aufgespalten. Dadurch wird ein dreidimensionales Netzwerk von miteinander verknüpften Polymerketten gebildet.

In der, z.B auf die beschriebene Art hergestellen Folie 2 werden zur Erzeugung der Poren 1 zunächst durchgehende Ionenspuren wie üblich hergestellt. Dazu wird die Folie 2 bzw. das membranförmige Polymer des vorstehenden Beispieles mit Xenon-Ionen von 13.0 MeV pro Nukleon spezifischer Energie durchstrahlt. Die Anzahl der Ionen pro cm² richtet sich nach den gewünschten Durchlaßeigenschaften der steuerbaren Membran bzw. des chemischen Ventiles. Dabei läßt sich die Trefferzahl zwischen einem Teilchen pro Werkstück bis zu ca. 10¹² Ionen pro cm² einstellen. Dementsprechend kann später im Ätzprozeß die Porengröße zwischen ca. 0.01 »m und ca. 100»m eingestellt werden. Im allgemeinen liegt die nominelle Porosität der Membran zwischen 1% und 50%, wobei diese nominelle Porosität = Teilchenzahl pro cm² mal Querschnittsfläche eines Loches ist.

Anschließend wird die Folie 2 in konzentrierter Natronlauge auf den gewünschten Ausgangsdurchmesser der Poren 1 aufgeätzt (zwischen 0.01»m und ca 100»m). Danach wird eine quellbare Gelschicht 3 der gewünschten Dicke durch Hydrolyse in einem wässrigen Ätzmedium bei geringer Konzentration und/oder geringen Temperatur des Ätzmediums auf der gesamten Oberfläche der Folie 2 und in den Poren 1 an deren Wänden erzeugt.

Der Chemismus der Gelbildung durch Behandlung eines beliebigen Polymers mit z.B. schwacher Natronlauge ist dabei wie folgt:

Polymere bestehen im allgemeinen aus kristallinen und amorphen Bereichen. Die Polymerketten schließen sich in den kristallinen Bereichen eng an die Nachbarketten an. Dadurch ist in diesen kristallinen Bereichen die Materialdichte erhöht und die Diffusion behindert. In den amorphen "glasartigen" Bereichen steht dagegen relativ viel freies Volumen für Diffusionsvorgänge zur Verfügung. Wasser und die hydratisierten Ionen der Natronlauge (Na+ und OH-) können in diese amorphen Bereiche eindringen und die Polymerketten schrittweise zerkleinern. Die Anlagerung von Wassermolekülen an die elektrisch geladenen Gruppen der chemisch geätzten Polymerketten entspricht einem makroskopisch beobachtbaren Quellvorgang (Gelbildung). Bei ausreichender Einwirkungsdauer des Ätzmediums entstehen kleine Bruchstücke des Polymers, die in Lösung gehen können (Solbildung).

Durch die beschriebene Hydrolisierung wird die Schicht 3 wasseraufnahmefähig gemacht. Die Schicht 3 besteht somit aus Polymerketten, die von Wasser durchsetzt sind, wobei die Kettenlänge kontinuierlich von der Matrix zu ihrer Oberfläche hin abnimmt. Die Dicke der Gelschicht 3 wird dabei auf den Durchmesser der Poren 1 derart abgestimmt, daß ein optimales Regelverhalten der Folie 2 als chemisches Ventil zustandekommt. Es ermöglicht z.B. eine dünne Gelschicht 3 ein ganz allmähliches Schließen der Poren 1 bei anfänglich großem Strömungsleitwert. Oder andererseits ermöglicht eine dicke Gelschicht 3 bei anfänglich niederem Strömungsleitwert ein sehr schnelles Verschließen des chemischen Ventiles als Funktion der Temperatur bzw. eines anderen externen Parameters wie der Konzentration von chemischen Stoffen oder dem pH-Wert. Bei dem eingangs genannten Beispiel quillt ein 100»m dicker Film in Wasser bei 0°C und schrumpft mit wachsender Temperatur zunehmend bis fast zur vollständigen Verdrängung des aufgenommenen Wassers aus der Polymermatrix bei ca. 60°C. Der Prozeß ist umkehrbar, wobei die Quellbarkeit mit zunehmenden Gehalt des Monomers B wächst. Die Gelschicht 3 bezieht somit ihre Eigenschaften vom Ausgangspolymer, von der Ätzbehandlung und von den Umgebungsbedingungen ihres Einsatzortes her.

Die Herstellung eines Polymerfilms nach der zweiten Ausführung, der aus mehreren Polymeren geschichtet oder zusammengesetzt ist, erfolgt auf ähnlichen Weise, jedoch in einer etwas anderen Reihenfolge:
Aus dem Polymer A allein wird zunächst eine Matrix 2 durch Bestrahlen und Ätzen, so wie bei der ersten Ausführung im vorstehenden beschrieben, erzeugt. Die Matrix 2 weist danach durchgehende Mikrolöcher 1 auf, die somit nur durch das Polymer A hindurchgehen. Anschließend wird die Matrix 2 in flüssiges oder gasförmiges Monomer B getaucht und zur Polymerisation von Monomer B auf der Matrix ionisierender Strahlung, wie z.B. UV-Licht, Elektronenstrahlung oder auch Gamma-Strahlen ausgesetzt. Die Dicke der Schicht wird im wesentlichen durch die Monomerkonzentration sowie durch weitere Parameter der Bestrahlung und die Temperatur bestimmt. Auf diese Weise erhält man eine Schicht, die entweder bereits selbst schon ausreichend responsiv ist, oder deren Responsibilität durch weiters Hydrolisieren und/oder Anheften weiterer chemischer Gruppen bzw. Moleküle gesteigert oder allgemein optimiert werden kann. Responsibilität bedeutet in diesen Zusammenhang die Fähigkeit, eine Schicht oder ein Material durch äußere Stimuli angeregt quellen oder schrumpfen zu lassen. Das letztgenannte Anheften zu diesem Zweck kann natürlich auch zusätzlich bei der weiter vorne beschriebenen ersten Ausführung des chemischen Ventiles erfolgen.

Generell kann als Ausgangsmaterial zur Herstellung eines chemischen Ventiles nach der Erfindung neben den eingangs genannten Beispielen jedes beliebige teilchenspurempfindliche Polymer oder Copolymer eingesetzt werden, vorzugsweise die organischen Polymere wie z.B Polycarbonate, Polyethylenterephthalate oder Polyimide.

## Patentansprüche

1. Chemisches Ventil mit einer porösen Matrix sowie einer durch ihre Umgebungsbedingungen stimuliert quellbaren Gelschicht, deren dadurch bedingte Kontraktionen oder Dilatationen die Poren in der Matrix öffnen oder verschließen, gekennzeichnet durch die folgenden Merkmale:
a) die Matrix ist eine Mikroporenmembran mit einer oder mehreren, auf vorbestimmten Durchmesser aufgeätzten und durch die Matrix durchgehenden parallelen Kernspuren und besteht
b) aus einem Copolymer aus zwei oder mehreren verschiedenen Monomertypen, wobei
c) mindestens ein Typ A gute Kernspureigenschaften bezüglich der Bildung von latenten Kernspuren nach einer Bestrahlung mit beschleunigten Teilchen und mindestens ein weiterer Typ B gute Quelleigenschaften in Abhängigkeit von der Temperatur und/oder von den Bedingungen in einer wässrigen Lösung oder durch ein anderes Stimulans aufweist.

2. Chemisches Ventil nach Anspruch 1, dadurch gekennzeichnet, daß die einzelnen Monomertypen A und B in der Matrix miteinander gemischt sind.

3. Chemisches Ventil nach Anspruch 1, dadurch gekennzeichnet, daß einzelne Polymere aus den Typen A und B schichtweise übereinander aufgebracht sind.

4. Chemisches Ventil nach Anspruch 3, dadurch gekennzeichnet, daß der Typ B erst nach der Bestrahlung und Aufätzung des Polymeres A in der Monomerphase an dieses angeheftet oder aufpolymerisiert ist und die Mikroporen der aufgeätzten Kernspuren somit nur durch das Polymer A hindurchgehen.

5. Chemisches Ventil nach Anspruch 1, dadurch gekennzeichnet, daß das Monomer A Diethylenglykol-bis-allylcarbonat ist.

6. Chemisches Ventil nach Anspruch 1, dadurch gekennzeichnet, daß das Monomer B aminosäurehaltig ist.

7. Chemisches Ventil nach Anspruch 6, dadurch gekennzeichnet, daß das Monomer B Methacryloyl-L-alalinmethylester ist.

8. Chemisches Ventil nach den Ansprüchen 2, 5, 6 oder 7 dadurch gekennzeichnet, daß die Monomere A und B im Verhältnis 80:20 gemischt sind.

9. Chemisches Ventil nach Anspruch 4, dadurch gekennzeichnet, daß das Polymer B die Mikroporen des Polymeres A auskleidet.

10. Chemisches Ventil nach einem der Ansprüche 3,4, oder 9, dadurch gekennzeichnet, daß auf oder in der Außenfläche des Polymers B weitere, chemisch oder thermisch stimulierbare Gruppen von Molekülen angeheftet sind.

11. Verfahren zur Erzeugung eines chemischen Ventiles nach Anspruch 1 oder 2 mit den folgenden Verfahrensschritten:
a) Bestrahlen eines membranförmigen Copolymers aus den gemischten Monomeren nach Anspruch 2 mit energiereichen Ionen und Erzeugen von durch das Copolymer durchgehenden Kernspuren,
b) Ätzen des Copolymers bis zur Erzeugung von durchgehenden Poren des gewünschten Durchmessers,
c) kurzzeitiges Hydrolisieren des Copolymers in einem wässrigen Ätzmedium geringer Konzentration und/oder geringer Temperaturänderung mit Erzeugen einer quellbaren Gelschicht auf dem Copolymer und/oder in den Poren.

12. Verfahren zur Erzeugung eines chemischen Ventiles nach Anspruch 1, 3, 4 oder 9 mit den folgenden Verfahrensschritten:
a) Bestrahlen eines membranförmigen Polymers A mit den besonderen Kernspureigenschaften nach Anspruch 1,c mit energiereichen Ionen und Erzeugen von den durch das Polymer durchgehenden Kernspuren,
b) Ätzen des Polymers bis zur Erzeugung von durchgehenden Poren des gewünschten Durchmessers,
c) Aufbringen des Types B mit den besonderen Quelleigenschaften nach Anspruch 1,c auf das membranförmige Polymer A.

13. Verfahren nach Anspruch 12 mit dem weiteren Verfahrensschritt:
d) Hydrolisieren des aufgeschichteten Copolymers aus A und B in einem wässrigen Ätzmedium geringer Konzentration und/oder geringer Temperatur mit Erzeugen einer quellbaren Gelschicht auf dem Copolymer und/oder in den Poren.

14. Verfahren nach Anspruch 11 oder 13, gekennzeichnet durch den weiteren Verfahrensschritt:
e) Anheften von weiteren chemisch oder thermisch stimulierbaren Molekülgruppen an die im Schritt d) erzeugte Hydrolyseschicht.

## Claims

1. Chemical valve, having a porous matrix and a gel layer, which is swellable when stimulated by its surrounding conditions and the contractions or dilations of which, caused thereby, open or close the pores in the matrix, characterised by the following features:
a) the matrix is a micropore diaphragm, having one or a plurality of parallel nuclear tracks, which are etched upon predetermined diameters and traverse the matrix, and comprising
b) a copolymer formed from two or more different monomer types,
c) at least one type A, having good nuclear track properties with respect to the formation of latent nuclear tracks after an irradiation treatment with accelerated particles, and at least one additional type B, having good swelling properties in dependence on the temperature and/or on the conditions in an aqueous solution or by another stimulant.

2. Chemical valve according to claim 1, characterised in that the individual monomer types A and B in the matrix are mixed together.

3. Chemical valve according to claim 1, characterised in that the individual polymers formed from types A and B are applied in layers above one another.

4. Chemical valve according to claim 3, characterised in that type B is only adhered to polymer A or polymerised thereon after said polymer has been irradiated and etched-on in the monomer phase, and the micropores of the etched-on nuclear tracks only, in consequence, pass through polymer A.

5. Chemical valve according to claim 1, characterised in that monomer A is diethylene glycol-bis-allyl carbonate.

6. Chemical valve according to claim 1, characterised in that monomer B contains amino acid.

7. Chemical valve according to claim 6, characterised in that monomer B is methacryloyl-L-alaline methyl ester.

8. Chemical valve according to claims 2, 5, 6 or 7, characterised in that the monomers A and B are mixed in the ratio 80:20.

9. Chemical valve according to claim 4, characterised in that polymer B covers the micropores of polymer A.

10. Chemical valve according to one of the claims 3, 4 or 9, characterised in that additional groups of molecules, which can be chemically or thermally stimulated, are adhered on or in the external surface of polymer B.

11. Method of producing a chemical valve according to claim 1 or 2 with the following method steps:
a) irradiating a diaphragm-like copolymer formed from the mixed monomers of claim 2 with energy-rich ions and producing nuclear tracks which traverse the copolymer,
b) etching the copolymer until transversing pores of the desired diameter are produced,
c) hydrolysing the copolymer for a short time in an aqueous etching medium of low concentration and/or low temperature change together with producing a swellable gel layer on the copolymer and/or in the pores.

12. Method of producing a chemical valve according to claim 1, 3, 4 or 9 with the following method steps:
a) irradiating a diaphragm-like polymer A with the special nuclear track properties of claim 1c with energy-rich ions and producing the nuclear tracks which traverse the polymer,
b) etching the polymer until transversing pores of the desired diameter are produced,
c) applying type B, having the special swelling properties of claim 1c, to the diaphragm-like polymer A.

13. Method according to claim 12 with the additional method steps:
d) hydrolysing the layered copolymer formed from A and B in an aqueous etching medium of low concentration and/or low temperature together with producing a swellable gel layer on the copolymer and/or in the pores.

14. Method according to claim 11 or 13, characterised by the additional method step:
e) adhering additional molecule groups, which can be chemically or thermally stimulated, to the hydrolytic layer produced in step d).

## Revendications

1. Vanne chimique comportant une matrice poreuse ainsi qu'une couche de gel gonflable stimulée par les conditions de son environnement, dont les contractions ou dilatations dues à ces phénomènes ouvrent ou ferment les pores de la matrice, grâce aux caractéristiques suivantes :
a) la matrice est une membrane à micropores avec une ou plusieurs traces nucléaires parallèles, attaquées chimiquement sur un diamètre, prédéterminée et traversant la matrice et elle se compose,
b) d'un copolymère constitué de deux ou de plusieurs types différents de monomères, dans lequel,
c) au moins un type A comporte de bonnes propriétés de traces nucléaires concernant la formation de traces nucléaires latentes après une irradiation avec des particules accélérées et au moins un autre type B comprend de bonnes propriétés de gonflement en fonction de la température et/ou des conditions dans une solution aqueuse ou grâce à un autre stimulant.

2. Vanne chimique selon la revendication 1, caractérisée en ce que les types de monomères individuels A et B sont mélangés ensemble dans la matrice.

3. Vanne chimique selon la revendication 1, caractérisée en ce que des polymères individuels des types A et B sont appliqués par couches les uns au-dessus des autres.

4. Vanne chimique selon la revendication 3, caractérisée en ce que le type B, tout de suite après l'irradiation et la corrosion du polymère A dans la phase monomère, est fixé sur celui-ci ou est polymérisé et les micropores des traces nucléaires attaquées chimiquement traversent donc seulement le polymère A.

5. Vanne chimique selon la revendication 1, caractérisée en ce que le monomère A est du diéthylèneglycol-bis-allylcarbonate.

6. Vanne chimique selon la revendication 1, caractérisée en ce que le monomère B contient de l'aminoacide.

7. Vanne chimique selon la revendication 6, caractérisée en ce que le monomère B est du méthacyloyl-L-alalineméthylester.

8. Vanne chimique selon les revendications 2, 5, 6 ou 7, caractérisée en ce que les monomères A et B sont mélangés dans le rapport 80:20.

9. Vanne chimique selon la revendication 4, caractérisée en ce que le monomère B revêt les micropores du polymère A.

10. Vanne chimique selon une des revendications 3, 4 ou 9, caractérisée en ce que sur ou dans la surface extérieure du polymère B sont fixés d'autres groupes de molécules stimulables chimiquement ou thermiquement.

11. Procédé de réalisation d'une vanne chimique selon les revendications 1 ou 2, avec les étapes suivantes de procédé :
a) irradiation d'un copolymère en forme de membrane composé de monomères mélangés selon la revendication 2 avec des ions de haute énergie et création de traces nucléaires traversant le copolymère,
b) corrosion du copolymère jusqu'à obtention de pores du diamètre souhaité,
c) hydrolyse de courte durée du copolymère dans un milieu corrosif aqueux de faible concentration et/ou de petite variation de température avec création d'une couche de gel gonflable sur le polymère et/ou dans les pores.

12. Procédé de réalisation d'une vanne chimique selon les revendications 1, 3, 4 ou 9, avec les étapes de procédé suivantes :
a) irradiation d'un copolymère A en forme de membrane ayant les propriétés de traces nucléaires particulières selon la revendication 1, c avec des ions de haute énergie et obtention des traces nucléaires traversant le copolymère,
b) corrosion du copolymère jusqu'à obtention de pores traversants du diamètre souhaité,
c) application du type B ayant les propriétés particulières de gonflement selon la revendication 1, c sur le polymère A en forme de membrane.

13. Procédé selon la revendication 12 avec l'autre étape de procédé suivante :
d) hydrolyse du copolymère empilé par couches à partir de A et de B dans un milieu corrosif aqueux de faible concentration et/ou de faible température avec création d'une couche de gel gonflable sur le copolymère et/ou dans les pores.

14. Procédé selon la revendication 11 ou 13, caractérisé par l'autre étape de procédé :
e) fixation d'autres groupes de molécules stimulables chimiquement ou thermiquement sur la couche hydrolysée obtenue à l'étape d).
